# EUROPEAN PATENT SPECIFICATION

(11) **EP 1 540 352 B1**
(45) Date of publication and mention of the grant of the patent: **13.12.2006**
(21) Application number: 03753693.5
(22) Date of filing: 19.09.2003
(51) Int. Cl.: G01N 33/66, G01N 33/74

(54) **GLYCATED INSULIN AS A BIOMARKER FOR DIAGNOSIS AND MONITORING OF DIABETES**
GLYKOLISIERTES INSULIN WIE BIOMARKER ZUR DIAGNOSE UND ÜBERWACHUNG VON DIABETES
INSULINE GLYQUEE EN TANT QUE BIOMARQUEUR POUR LE DIAGNOSTIC ET LE CONTROLE DU DIABETE

(30) Priority: 19.09.2002 GB 0221686
(43) Date of publication of application: 15.06.2005
(73) Proprietor: Uutech Limited, Coleraine, County Londonderry BT52 1ST (GB)
(72) Inventor: MCKILLOP, Aine, M., Co. Antrim Northern Ireland BT54 6JA (GB); O'HARTE, Finbarr, P., M., Co. Londonderry Northern Ireland BT55 7S (GB); FLATT, Peter, R., Co. Antrim Northern Ireland (GB)
(74) Representative: O'Connell, Maura
(86) International application number: PCT/GB2003/004128
(87) International publication number: WO 2004/027430

(56) References cited:
- US-A- 5 733 546
- MCKILLOP A M ET AL: "Production and characterization of specific antibodies for evaluation of glycated insulin in plasma and biological tissues" JOURNAL OF ENDOCRINOLOGY, vol. 167, no. 1, October 2000 (2000-10), pages 153-163, XP002266967 ISSN: 0022-0795
- MCKILLOP A M ET AL: "Evaluation of glycated insulin in diabetic animals using immunocytochemistry and radioimmunoassay" BIOCHEMICAL AND BIOPHYSICAL RESEARCH COMMUNICATIONS, vol. 286, no. 3, 24 August 2001 (2001-08-24), pages 524-528, XP002266968 ISSN: 0006-291X
- LINDSAY J R ET AL: "Demonstration of increased concentrations of circulating glycated insulin in human Type 2 diabetes using a novel and specific radioimmunoassay." DIABETOLOGIA, vol. 46, no. 4, April 2003 (2003-04), pages 475-478, XP002266969 ISSN: 0012-186X

## Description

### Field of the Invention

The present invention relates to the use of a novel biomarker for the prediction and management of diabetes mellitus together with its complications. More particularly, the present invention provides methods for predicting the onset of diabetes and for monitoring disease progression.

One of the major pathophysiological consequences of long term elevation of plasma glucose in diabetes is an increase in the non-enzymatic glycation (glycosylation) of proteins.

Formation of advanced glycation end-products (AGEs) plays an important role in the long-term metabolic consequences of diabetes, including ophthalmic, renal and atherosclerotic vascular complications. Glycation has been shown also to interfere with normal cellular functions including the activities of various enzymes such as Cu-Zn superoxide dismutase.

Insulin is known to be glycated in the pancreatic beta cells under conditions of hyperglycaemia. Thus glycation results in an impairment of insulin action.

Glycated insulin exhibits a reduced ability to regulate plasma glucose homeostasis in vivo and to stimulate adipose tissue lipogenesis and / or glucose uptake and oxidation by isolated diaphragm and abdominal muscle in vitro.

The site of glycation of human insulin has been identified by electrospray tandem mass spectrometry as the N-terminal Phe¹ of the B-chain.

### Summary of the invention

It has long been accepted that, in diabetes, the concentration of glycated proteins increases over time, with disease progression and with increased blood glucose concentration. For example, the glycation of proteins such as haemoglobin has been shown to increase as levels of blood glucose increase in accordance with the severity and duration of diabetes.

It has long been considered that, in a similar way, the concentration of glycated insulin in the blood increases with increasing blood glucose in diabetes.

Surprisingly, the present inventors have shown that, contrary to expectations and the teaching of the prior art, the concentration of glycated insulin decreases with increased disease severity and / or duration of diabetes.

Thus, according to a first aspect of the present invention there is provided an in vitro method of monitoring the progression of diabetes from a first timepoint to a later timepoint, said method comprising the steps:
measuring the concentration of glycated insulin in a first biological sample obtained at a first time point,
measuring the concentration of glycated insulin in a second biological sample, obtained at a later time period than the first biological sample,
determining the difference in concentration of glycated insulin between the first and second biological samples, wherein a lower concentration at the second timepoint is indicative of increased disease severity and/or loss of control of blood glucose.

Throughout this specificaton, references to glycated insulin should be understood to refer to an insulin molecule, a proinsulin molecule or fragments of insulin or proinsulin linked to one or more molecules of glucose, metabolites or related reducing sugars in the form of glucitol adducts, Schiff base adducts or amadori products.

Moreover, the demonstration that glycated insulin concentrations are greater in early stage/well-controlled diabetes than in later stage/poorly controlled diabetes enables the use of glycated insulin as an early indicator of diabetes. In particular, the inventors' work suggests that glycated insulin may be used in the early diagnosis of diabetes e.g. prior to the appearance of other symptoms and/or diagnostic markers such as hyperglycaemia. Thus glycated insulin may be used in diagnosis of diabetes prior to the appearance of symptoms such as blood glucose levels in excess of the normal range.

Prediabetes has been defined as the stage before the development of diabetes, with normal glucose tolerance but with an increased risk of developing diabetes at some future time. Examples of increased risk might include family history of diabetes, prior diagnosis of gestational diabetes, presence of a positive antibody test for diabetes, or insulin resistance.

More recently, the American Diabetes Association has suggested a new definition: "Pre-diabetes is the state that occurs when a person's blood glucose levels are higher than normal but not high enough for a diagnosis of diabetes...Doctors sometimes refer to this state of elevated blood glucose levels as Impaired Glucose Tolerance or Impaired Fasting Glucose (IGT/IFG), depending on which test is used to detect it." Thus the criteria for prediabetes, although not clearly defined, can be considered to mean that an individual is on track to attain the diagnostic criteria in the future.

Thus, in a second aspect, the present invention provides an in vitro method of early diagnosis of diabetes or a predisposition to diabetes in an individual, the method comprising the steps: measuring the concentration of glycated insulin in the biological sample, obtained from an individual in which glucose levels are less than 11.1 mmol/l wherein the presence of glycated insulin at a concentration greater than a predetermined minimum is indicative of the presence of diabetes or predisposition to diabetes.

In the studies provided herein, as well as studying the average levels of glycated insulin in control subjects and diabetic subjects, the inventors also examined the individual data for each subject.

This clearly suggests the invention may be further used to predict the onset of diabetes and/or identify individuals with a predisposition to diabetes in advance of the appearance of any symptoms conventionally used in the diagnosis of the disease. Thus, the invention may be used in the testing and monitoring of individuals believed to be at risk of developing diabetes, e.g. individuals with a family history of the disease in order to enable early intervention to prevent onset or development of the disease. Such testing and monitoring may thus be used to identify or predict the onset of diabetes weeks or months in advance of the onset of the disease.

The method allows prediction of the onset of diabetes in an individual.

As described above, the methods of the invention may be used prior to the appearance of symptoms commonly used in the diagnosis of diabetes. Thus, in the second aspect of the invention, the biological sample has a glucose concentration within a normal range i.e. a range not normally considered diagnostic of diabetes, for example less than 11.1 mmol/l (20mg/dl).

In preferred embodiments, the biological sample has a glucose concentration for a random plasma sample preferably less than 10 mmol/l, even more preferably less than 9 mmol/l, most preferably less than 8 mmol/l. Alternatively, the biological sample has a glucose concentration in a fasting plasma sample less than 7.0 mmol/l (126 mg/dl).

In preferred embodiments of the invention the predetermined minimum concentration of glycated insulin is at least 20 pmol/l, more preferably at least 23 pmol/l, even more preferably at least 25 pmol/l, most preferably at least 28 pmol/l.

Thus in preferred embodiments preferably glycated insulin is considered to be raised if it is greater than 20 pmol/l, preferably greater than 25 pmol/l, more preferably greater than 28 pmol/l and even more preferably greater than 30 pmol/l.

In preferred embodiments blood component concentrations, e.g. glucose or glycated insulin are measured in non fasted plasma.

In certain preferred embodiments of the second and third aspects of the invention, the predetermined minimum concentration is the concentration of glycated insulin measured in a sample from the same individual at an earlier timepoint. Thus these aspects may be used in the monitoring of disease progression and/or beta cell damage/dysfunction in an individual.

Without being limited to any one theory, the early elevation in glycated insulin levels may be indicative of early stage dysfunction of or damage to pancreatic beta cells, prior to significant elevation of blood glucose. Thus, the invention further provides a method of evaluating the competency of the pancreatic beta cells of an individual, the method comprising the step of providing a biological sample, (e.g. blood, serum or plasma) from the individual, and determining the level of glycated insulin in the sample, wherein the presence of glycated insulin in the sample at a concentration in excess of a predetermined minimum is indicative of dysfunction of or damage to the beta cells.

Prediction of the onset of the disease permits early counselling and intervention. Early detection of the disease enables patient treatment and management at an early stage.

In the invention, the presence and quantification of glycated insulin in the sample may be carried out using any assay known in the art. For example, such assays may include, but are not limited to, ELISA assays, or radioimmunoassay (RIA).

Other preferred methods of detecting the presence of and quantifying glycated insulin includes IRMA (immunoradiometric) assays and mass spectrometry techniques. The procedures for carrying out such assays are known in the art.

In a third aspect, the invention provides the use of glycated insulin in vitro as a predictive marker for glucose intolerance and/or diabetes.

In a fourth aspect, the invention provides the use of glycated insulin in vitro as a predictive marker for prediabetes or to predict the onset of diabetes.

Glucose intolerance is considered to be the stage prior to overt diabetes in that a random test of glucose concentration may not give a value high enough to fall within the diabetes diagnostic criterion. Prediabetes is a stage even before a clear demonstration of glucose intolerance.

There is described an in vitro assay method for detecting the presence of glycated insulin in a biological sample, in which glucose levels are normal, said assay method comprising the steps:
providing a biological sample; measuring the concentration of glycated insulin in the biological sample;
wherein the presence of glycated insulin at a concentration greater than a predetermined minimum is indicative of diabetes or predisposition to diabetes.

There is described an assay kit for carrying out a method of the invention; said kit comprising at least one antibody with binding specificity to glycated insulin, means to detect binding of the at least one antibody to glycated insulin, details of a concentration range of glycated insulin considered to be elevated from normal levels , and instructions on how the assay is to be performed and how the results are to be interpreted.

Preferably, the instructions are such that the finding of a decrease in glycated insulin levels from a first sample obtained from a patient to a later sample obtained from the patient is indicative of increased disease severity and/or loss of glucose control.

There is described the use of any of the above methods to diagnose if a patient is glucose intolerant.

In one embodiment glycated insulin is part of an assay for a range of metabolites and substrates relating to a number of diseases to determine the disease status of individuals.

There is described the use of glycated insulin to monitor and record diabetic status.

The invention will now be described, by way of example only, with reference to the accompanying figures, wherein:
Figure 1 shows glycated haemoglobin (HbA_{1c}), plasma glucose and glycated insulin concentrations of control subjects and diabetic patients exhibiting good, moderate or poor metabolic control. Characteristics and numbers in each group are given in Table 1. Values are mean ± SEM. ****p*<0.001 compared with control subjects.
Figure 2 shows individual glycated insulin concentrations of 75 of the control non diabetic subjects showing 4 individuals with levels in excess of 2 standard deviations from the overall group mean - other features of this group are provided in Table 1 and figure 1.

### EXAMPLE 1

Mid-morning blood samples were withdrawn from type 2 diabetic subjects (n=102) attending routine hospital review appointments. All subjects were controlled by diet alone or by oral hypoglycaemic agents. No subjects were receiving insulin. Type 2 diabetic subjects were divided into 3 groups depending upon their glycaemic control. Group 1 represented subjects under good glycaemic control with a HbA_{1c} < 7% (upper limit of non-diabetic range, 6.5%). Group 2 was comprised of type 2 diabetic subjects with moderate glycaemic control (HbA_{1c} 7-9 %) and Group 3 represented subjects with a HbA_{1c} > 9% (poor glycaemic control). Age- and sex-matched normal healthy individuals served as controls. This study was approved by the ethics committee and carried out following informed consent from all subjects.

A specific RIA for glycated insulin has been developed. In brief, an N-terminally glycated synthetic insulin peptide, closely related to the amino-terminal sequence of the insulin B-chain (Phe-Val-Asn-Gln-His Leu-Tyr-Lys) was used to raise specific antibodies in rabbits and guinea pigs. This peptide comprised the naturally occurring 1-6 sequence of insulin B-chain with a Tyr and Lys substituted at positions 7 and 8, respectively. For determination of glycated insulin, the insulin peptide was glycated under hyperglycaemic reducing conditions and iodinated using the solid phase iodogen method, generating a high specific activity mono-iodinated I¹²⁵-tyrosylated glycated peptide tracer. A glycated insulin antiserum was used to establish a dextran-coated charcoal RIA with a glycated human insulin standard curve in the presence of insulin free serum. The glycated insulin antibody cross-reacted 56% with glycated proinsulin but cross-reaction with non-glycated insulin, proinsulin and other pancreatic hormones was negligible. Serum insulin was determined using a routine radioimmunoassay with human insulin standard. Glucose concentrations were determined using the glucose oxidase method and HbA_{1c} was measured in whole blood by ion-exchange HPLC. Serum creatinine was determined using a multilayered dry slide aminohydrolase technique.

Data are expressed as mean ± SEM. Significant differences between groups of data were assessed using the unpaired Student's t test and statistical significance was assumed if *p*<0.05.

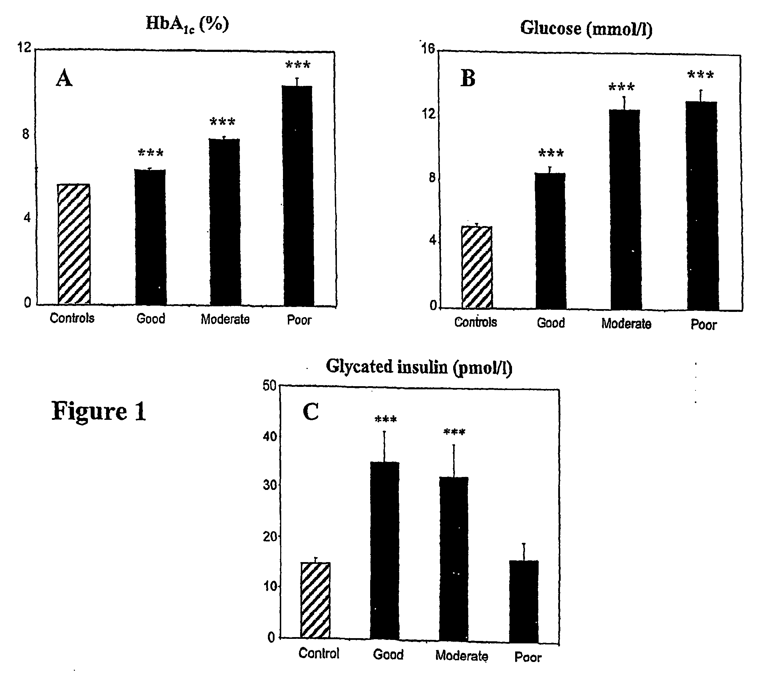

Figure 1 illustrates that glycated insulin concentrations of diabetic patients exhibiting good and moderate metabolic control are raised relative to HbA_{1c}, plasma glucose and healthy control subjects.

The characteristics of the 75 control subjects and 102 diabetic patients are summarised in Table 1. Control and diabetic groups were well matched for age and sex. Serum creatinine concentrations were similar, indicating freedom from renal disease. BMI of diabetic groups was increased compared with controls. Duration of diabetes increased progressively in good, moderate and poorly controlled groups. A total of 70 diabetic subjects were taking oral hypoglycaemic agents of whom 16 were taking metformin alone, 27 sulphonylurea alone and 22 on a combination of metformin and sulphonylurea. The remaining patients were treated with dietary restriction alone (31%) or a combination of other treatments (5%), which included acarbose. The distribution of patients in each group on these treatment regimes is given in Table 1.

Insulin concentrations measured in the control, good, moderate and poorly controlled groups were 148 ± 19, 191 ± 25, 225 ± 40 and 159 ± 40 pmol/l, respectively.

Control subjects had mean glycated haemoglobin values of 5.7 ±.0.1% compared with 6.4 ± 0.1 (*p*<0.001), 7.9 ± 0.1 (*p*<0.001) and 10.4 ± 0.4% (*p*<0.001) for good, moderate and poorly controlled diabetic groups, respectively (Figure 1, graph A). Plasma glucose concentrations were similarly raised in the diabetic groups, with highest values observed in the poorly controlled group (Figure 1, graph B). A positive correlation between glycated haemoglobin and glucose concentration was evident in the combined groups (*r*=0.322; *p*<0.01).

As shown in Figure 1, graph C, plasma glycated insulin concentration of good and moderately controlled diabetic groups were increased 2.4-fold (*p*<0.001) and 2.2-fold (*p*<0.001) compared with control subjects. Plasma glycated insulin concentrations in the control group, well, moderately and poorly controlled diabetic groups were 12.8 ± 1.1 (SD 9.2), 29.8 ± 5.4 (SD 35.7), 27.3 ± 5.7 (SD 36.5) and 13.5 ± 2.9 (SD 11.9) pmol/l, respectively. Glycated insulin concentrations were higher in the well controlled group with the lowest glucose concentration and the shortest duration of diabetes. This is surprising as increased glucose levels are thought to promote glycation of proteins. Thus, as observed for glycated haemoglobin, the amount of glycated insulin would be expected to increase in keeping with a time and concentration dependent process in the pancreatic beta cell as disclosed in the art.

The unexpected observation of a higher concentration of glycated insulin in the test group with the lowest glucose concentration led the inventors to conclude that glycated insulin is an early marker of diabetes and its potential complications.

### Example 2

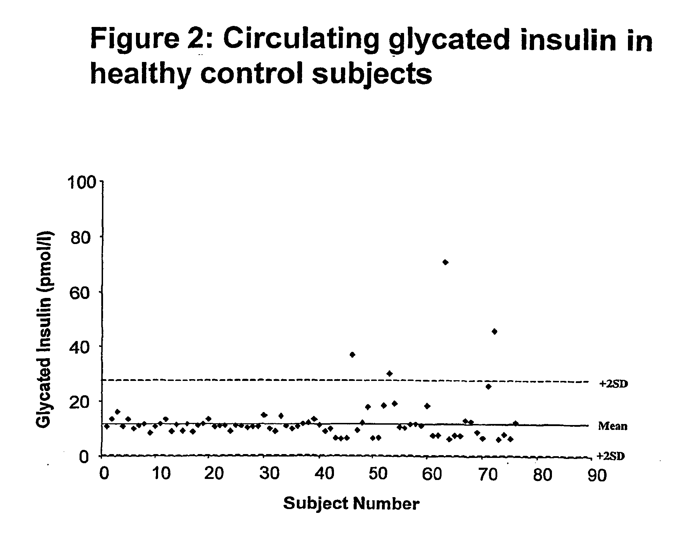

The individual data for each subject was examined. The results are shown in Figure 2. As can be seen, surprisingly, the inventors discovered that in the control subjects, all of whom had been classified as non-diabetic using conventional criteria, (glucose concentrations of control subjects were within normal range of glucose concentration) there were four out of the seventy-five subjects who nevertheless displayed glycated insulin concentration in excess of two standard deviations of the overall mean. This interestingly correlates with the incidence of the diabetes in the population at large, which is between 3-5%. The inventors conclude that as one would expect that 2-4 individuals in the population size used in the study would go on to develop diabetes that this provides evidence that increased glycated insulin concentration is indicative of prediabetes.

The results clearly show that measurement of glycated insulin in non-diabetic subjects, in which glucose is not significantly raised, can be used to predict the onset of diabetes and/or identify individuals with a predisposition to diabetes in advance of the appearance of any symptoms conventionally used in the diagnosis of the disease.

Glycated insulin is suggested to be a feature of beta cell dysfunction and insulin resistance in diabetes.

Furthermore, glycated insulin is suggested to act as an insulin receptor antagonist.

Unlike for example glycated haemoglobin levels, the concentration of glycated insulin measured correlate inversely with the degree of hyperglycaemia and most importantly the duration of diabetes. This surprising observation indicated that elevation of glycated insulin/proinsulin is an early feature of beta cell dysfunction and diabetes occurring before increased glucose concentration and thus glycated insulin can be used as a prediction method of diabetes.

Glycated insulin concentration is dependent not only on the glycaemic environment but also the ability of the beta cells to transport and metabolise glucose to reactive phosphorylated forms at the intracellular sites of (pro)insulin synthesis and storage.

Additionally, the secretory activity and competency of the beta cells together with metabolic clearance rate will also determine the rates of delivery and removal of glycated insulin from the circulation. It is notable that these various parameters are deranged in prediabetes and in individuals with increased risk of developing diabetes.
It is proposed that beta cell competency is a determinant of the total plasma concentration of glycated insulin in individual patients depending on the status of their current disease.

A typical pattern of loss of beta cell competency, followed by increased concentration of glycated insulin, increased glucose concentration and loss of first phase glucose-induced insulin secretion followed by progressively impaired second-phase insulin secretion, with complications for example glucose blindness, and disproportionate hyperproinsulinaemia with impaired basal or steady state insulin secretion is proposed.

It is known that glucose concentration does not correlate with the incidence of complications, for example glucose blindness, which are well known in the art. It is proposed that measurement of glycated insulin provides an indicator of the progression of diabetes, which can be used to determine if complications are likely to arise.

Glycated insulin/proinsulin therefore provides a novel and potentially important biomarker with potential in the prediction, and management of diabetes together with its complications.

Although the present invention has been particularly shown and described with reference to a particular example, it will be understood by those skilled in the art that various changes in the form and details may be made therein without departing from the scope of the present invention.

**Table 1: Characteristics of controls subjects and type 2 diabetic patients.**

| | Control subjects | Patients with good control | Patients with moderate control | Patients with poor control |
|---|---|---|---|---|
| Number of subjects | 75 | 44 | 41 | 17 |
| Male/female ratio | 31/44 | 21/23 | 24/17 | 9/8 |
| Age (years) | 59.5 ± 2.1 | 64.6 ± 1.4 | 63.4 ± 1.7 | 61.0 ± 3.7 |
| Duration of diabetes (years) | -- | 4.8 ± 0.6 | 7.1 ± 0.9 | 8.5 ± 1.3 |
| BMI (kg/m²) | 25.6 ± 0.5 | 29.6 ± 1.0*** | 30.6 ± 1.0*** | 28.9 ± 1.3** |
| Serum Creatinine (µmol/l) | 86.0 ± 4.0 | 89.2 ± 3.6 | 89.0 ± 4.0 | 85.8 ± 3.6 |
| Diet (%) | --- | 40.9 | 26.8 | 17.6 |
| Metformin (%) | --- | 18.2 | 14.6 | 11.8 |
| Sulphonylureas (%) | --- | 29.5 | 26.8 | 17.6 |
| Metformin/ Sulphonyureas (%) | --- | 6.8 | 26.8 | 47.1 |
| ^{†}Other drug combinations (%) | --- | 4.6 | 4.9 | 5.9 |

| | | | | |
|---|---|---|---|---|
| Values are Mean ± SEM. ***p*<0.01, ***p*<0.001 compared with control subjects. ^{†}Other treatment combinations included metformin and acarbose, gliclazide and acarbose, and glibenclamide and acarbose. | | | | |

## Claims

1. An *in vitro* method of monitoring the progression of diabetes from a first timepoint to a later timepoint, said method comprising the steps,
measuring the concentration of glycated insulin in a first biological sample obtained at a first time point,
measuring the concentration of glycated insulin in a second biological sample obtained at a later timepoint than the first biological sample,
determining the difference in concentration of glycated insulin between the first and second biological samples, wherein a lower concentration at the second timepoint is indicative of increased disease severity and/or loss of control of blood glucose.

2. An *in vitro* method of early diagnosis of diabetes or a predisposition to diabetes in an individual, the method comprising the steps,
measuring the concentration of glycated insulin in a biological sample obtained from an individual in which glucose levels are less than 11.1 mmol/l,
wherein the presence of glycated insulin at a concentration greater than a predetermined minimum is indicative of the presence of diabetes or predisposition to diabetes.

3. The method according to claim 2 wherein said predetermined minimum concentration is the concentration of glycated insulin measured in a sample from the same individual at an earlier timepoint.

4. The method according to any one of claims 2 or 3 wherein said predetermined minimum concentration of glycated insulin in a non fasted sample is at least 20 pmol/l.

5. A method as claimed in any preceding claim wherein glycated insulin in the sample is measured by means of radioimmunoassay (RIA).

6. A method as claimed in any of claims 1 to 4 wherein the glycated insulin in the sample is measured by means of enzyme-linked immunosorbent assay (ELISA).

7. Use of glycated insulin *in vitro* as a predictive marker for glucose intolerance and/or diabetes.

8. Use of glycated insulin *in vitro* as a predictive marker for prediabetes or to predict the onset of diabetes.

## Patentansprüche

1. In-vitro-Verfahren zur Überwachung des Fortschreitens von Diabetes von einem ersten Zeitpunkt bis zu einem späteren Zeitpunkt, genanntes Verfahren umfasst die Schritte:
Messen der Konzentration von glykiertem Insulin in einer ersten biologischen Probe, die zu einem ersten Zeitpunkt erhalten wird,
Messen der Konzentration von glykiertem Insulin in einer zweiten biologischen Probe, die zu einem späteren Zeitpunkt als die erste biologische Probe erhalten wird,
Bestimmen des Konzentrationsunterschieds von glykiertem Insulin zwischen der ersten und der zweiten biologischen Probe, wobei eine niedrigere Konzentration zu dem zweiten Zeitpunkt auf erhöhte Krankheitsschwere und/oder Verlust der Kontrolle von Blutglucose hindeutet.

2. *In-vitro*-Verfahren der frühen Diagnose von Diabetes oder einer Disposition für Diabetes in einem Individuum, das Verfahren umfasst die Schritte:
Messen der Konzentration von glykiertem Insulin in einer biologischen Probe, die von einem Individuum erhalten wird, in der Glucosespiegel niedriger als 11,1 mmol/l sind,
worin die Gegenwart von glykiertem Insulin bei einer Konzentration, die größer als ein vorherbestimmtes Minimum ist, auf die Gegenwart von Diabetes oder der Disposition für Diabetes hindeutet.

3. Verfahren nach Anspruch 2, worin genannte vorherbestimmte Minimumkonzentration die Konzentration von glykiertem Insulin ist, die in einer Probe vom selben Individuum zu einem früheren Zeitpunkt gemessen wurde.

4. Verfahren nach einem der Ansprüche 2 oder 3, worin genannte vorherbestimmte Minimumkonzentration von glykiertem Insulin in einer Nicht-Nüchtern-Probe mindestens 20 pmol/l beträgt.

5. Verfahren nach einem der vorstehenden Ansprüche, worin glykiertes Insulin in der Probe mit Hilfe vom Radioimmunassay (RIA) gemessen wird.

6. Verfahren nach einem der Ansprüche 1 bis 4, worin das glykierte Insulin in der Probe mit Hilfe vom Enzyme Linked Immunosorbent Assay (ELISA) gemessen wird.

7. Verwendung von glykiertem Insulin *in vitro* als einen prädiktiven Marker für Glucoseintoleranz und/oder Diabetes.

8. Verwendung von glykiertem Insulin *in vitro* als einen prädiktiven Marker für Prädiabetes oder zur Vorhersage des Ausbruchs von Diabetes.

## Revendications

1. Méthode *in vitro* de contrôle de l'évolution du diabète à partir d'un premier instant jusqu'à un instant ultérieur dans le temps, ladite méthode comprenant les étapes consistant à :
mesurer la concentration en insuline glyquée dans un premier échantillon biologique obtenu à un premier instant,
mesurer la concentration en insuline glyquée dans un deuxième échantillon biologique obtenu à un instant postérieur à celui du premier échantillon biologique,
déterminer la différence, en termes de concentration en insuline glyquée, entre les premier et deuxième échantillons biologiques, dans laquelle une concentration inférieure au deuxième instant est indicatrice d'une gravité accrue de la maladie et/ou d'une perte de contrôle de la glycémie.

2. Méthode *in vitro* de diagnostic précoce du diabète ou d'une prédisposition au diabète chez un individu, la méthode comprenant l'étape consistant à :
mesurer la concentration en insuline glyquée dans un échantillon biologique obtenu chez un individu chez qui les taux de glucose sont inférieurs à 11,1 mmol/l,
dans laquelle la présence d'insuline glyquée à une concentration supérieure à un minimum prédéterminé est indicatrice de la présence du diabète ou d'une prédisposition au diabète.

3. Méthode selon la revendication 2, dans laquelle ladite concentration minimale prédéterminée est la concentration en insuline glyquée mesurée dans un échantillon provenant du même individu à un instant plus tôt.

4. Méthode selon l'une quelconque des revendications 2 ou 3, dans laquelle ladite concentration minimale prédéterminée en insuline glyquée dans un échantillon non à jeun est d'au moins 20 pmol/l.

5. Méthode selon l'une quelconque des revendications précédentes, dans laquelle l'insuline glyquée dans l'échantillon est mesurée à l'aide d'un radioimmunodosage (RIA).

6. Méthode selon l'une quelconque des revendications 1 à 4, dans laquelle l'insuline glyquée dans l'échantillon est mesurée à l'aide d'un dosage d'immuno-absorption enzymatique (ELISA).

7. Utilisation d'insuline glyquée *in vitro* comme marqueur de prédiction pour l'intolérance au glucose et/ou le diabète.

8. Utilisation d'insuline glyquée *in vitro* comme marqueur de prédiction pour le prédiabète ou pour prédire le début du diabète.
